# EUROPEAN PATENT SPECIFICATION

(11) **EP 1 818 071 B1**
(45) Date of publication and mention of the grant of the patent: **10.06.2009**
(21) Application number: 07250489.7
(22) Date of filing: 07.02.2007
(51) Int. Cl.: A61M 25/01, A61M 25/00

(54) **A medical apparatus, tubular insertion device and a tubular insertion device having the same medical apparatus**
Medizinische Vorrichtung, röhrenförmiges Einführgerät und röhrenförmiges Einführgerät mit der medizinischen Vorrichtung
Appareil médical, dispositif d'insertion tubulaire et dispositif d'insertion tubulaire doté dudit appareil médical

(30) Priority: 14.02.2006 JP 2006037162
(43) Date of publication of application: 15.08.2007
(73) Proprietor: ASAHI INTECC CO., LTD., Nagoya-shi, Aichi-ken (JP)
(72) Inventor: Shimogami, Manabu, Nagoya-shi Aichi-ken (JP); Goto, Shinichi, Nagoya-shi Aichi-ken (JP); Nihonmatsu, Masaaki, Nagoya-shi Aichi-ken (JP)
(74) Representative: Price, Nigel John King

(56) References cited:
- WO-A-93/18817
- JP-A- 2007 068 970
- US-A- 4 619 263
- US-A- 5 114 403

## Description

The invention relates to a medical apparatus, tubular insertion device and tubular insertion device having the medical apparatus which is attached to the tubular insertion device upon inserting it into a human body.

Upon therapeutically diagnosing human organs especially such as the blood vessel, the digestive tract, the urethral canal or the like, a tubular insertion device (a.k.a., tubular lumen insertion device) is used which has a flexible and axially elongate body. The operator who inserts the insertion device into the tract of the human body operates the insertion device at the proximal side so as to steer the distal end portion into the tract. The insertion device is represented by a catheter, a guide wire, a balloon catheter or the like.

When using the tubular insertion device, the operator may rotate the insertion device around its axis at the proximal side of a medical device. This procedure is required, for example, when operating the insertion device through tortuous tract, steering the preshaped insertion device or torsionally inserting the insertion device through an occlusion area of the tract.

When the insertion device pushes its distal end portion against the occlusion area or vascular wall upon rotating the insertion device around its axis, there is a possibility that the insertion device is subjected to a surplus rotational torque.

If the rotational torque reaches a magnitude more than a predetermined value, the insertion device may be torsionally deformed to sustain the buckling during the operating process. In this case, the operator has to withdraw the deformed insertion device from the tract and to replace the insertion device with the a new one. This would make patients burdensome who are therapeutically treated with the insertion device.

In order to solve the above disadvantages, for example, Japanese Patent Application Laid-open Publication No. 2005-296078 discloses such an insertion device having a diameter-reduced proximal at a proximal part which preferentially sustains the torsional buckling so as not to induce the buckling inside the tract. In addition, this document also discloses a structure which enables the operator to visually confirm the buckling inside the tract.

The insertion device which occurred the buckling may has to replace the new insertion device with a new one. And the operator may has to visually confirm an emergence of the buckling.

The buckling often requires the replacement of the insertion device once the operator visually confirm the buckling. This makes it difficult for the operator to instantaneously perceive the torque more than the predetermined value. Due to the difficulty of instantaneously perceiving the excessive torque, it may be time-consuming to visually confirm the buckling, which resultantly may delay the replacement of the insertion device so as to prolong the therapeutic operation.

In the meanwhile, Japanese Patent Application Laid-open Publication No. 10-66696 discloses a medical equipment which has a torque regulation device to control a catheter in which a rotary section is built in. It controls the torque transmission needed to drive the rotary section, but has no structure which enables the operator to instantaneously perceive that the insertion device sustains the torque more than the predetermined value during the use of the insertion device.

WO 93/18817 discloses a turn limiter for a catheter configured such that a torque may be applied to a torque element at a proximal end of the catheter by turning a turn knob until an allowable number of turns is completed.

Therefore, it is an object of the invention to overcome the above drawbacks, and provide a structure which is capable to obviate the necessity of replacing a tubular insertion device by protecting the insertion device against the buckling.

It is another object of the invention to provide a medical equipment (apparatus), tubular insertion device and tubular insertion device having the medical equipment which enables an operator to instantaneously perceive at the proximal side that the insertion device sustains the torque more than the predetermined value during the use of the insertion device.

According to the invention, there is provided a medical apparatus comprising: a main body provided to be connected to a tubular insertion device which is to be inserted inside a human body; and a cylindrical body provided in contact with said main body and arranged integrally to rotate said main body with said tubular insertion device; characterised by: a torque regulating mechanism located at a contact portion between said main body and said cylindrical body; said torque regulating mechanism provided to rotationally displace said cylindrical body to said main body at the contact portion to release an integral rotation between said cylindrical body and said main body so as to block a torque transmission to said tubular insertion device when more than a predetermined torque is applied to said cylindrical body.

With the torque applied to the cylindrical body more than the predetermined value, the torque releases the cylindrical body from the main body, thereby making the insertion device incapable to rotate so as to protect the insertion device against the torsional buckling. With the use of the medical equipment, it is possible to release a two-side contact which enables the operator to an integral rotation between the main body and the cylindrical body, thereby making it possible for the operator to instantaneously perceive at the proximal side that the insertion device sustains the torque more than the predetermined value.

Preferred embodiments of the present invention are illustrated in the accompanying drawings, in which:
Fig. 1 is a side elevational view of a medical equipment and an occlusion catheter of an example of an insertion device, to which the medical equipment is attached according to a first embodiment of the invention;
Fig. 2 is a cross-sectional view of the insertion device along the line A-A of Fig. 1;
Fig. 3 is a cross-sectional view of the insertion device along the line B-B of Fig. 1 ;
Fig. 4 is a cross-sectional view of the insertion device along the line C-C of Fig. 1;
Fig. 5 is an enlarged latitudinal cross-sectional view showing a connection area between the occlusion catheter and the medical equipment;
Fig. 6 is a side elevational view of the medical equipment;
Fig. 7 is a plan view of a front end surface of the medical equipment;
Fig. 8 is a longitudinal cross-sectional view of the medical equipment;
Fig. 9 is an enlarged perspective view of the medical equipment when subjected to a torque less than a predetermined value exclusive;
Fig. 10 is an enlarged perspective view of the medical equipment when subjected to a torque more than the predetermined value inclusive;
Fig. 11 is an explanatory view showing a relationship between body-side tooth portions and cylinder-side tooth portions;
Fig. 12 is a side elevational view of the medical equipment according to a second embodiment of the invention;
Fig. 13 is a longitudinal cross-sectional view of the medical equipment;
Fig. 14 is an explanatory view showing a relationship between the body-side tooth portions and the cylinder-side tooth portions;
Fig. 15 is a side elevational view of the medical equipment and the occlusion catheter, to which the medical equipment is attached according to a third embodiment of the invention;
Figs. 16-18 are views showing the meshing relationship between the slope surfaces of the body-side tooth portions and the cylinder-side tooth portions (modification forms);
Fig. 19 is a side elevational view of the medical equipment, to which the occlusion catheter is attached (modification form);
Fig. 20 is a longitudinal cross-sectional view of the medical equipment, to which the occlusion catheter is attached (modification form); and
Fig. 21 is a cross-sectional view of a chuck along the line J-J of Fig. 20 (modification form).

In an embodiment of the invention, there is provided a medical equipment which is attached to a tubular lumen insertion device having a flexible and axially elongate body. The tubular lumen insertion device has a distal end portion which is to be inserted into the tract of the human body, and having a proximal end, to which an operative portion is secured. The medical equipment has a main body, a cylindrical body, and a torque regulation mechanism. The main body is connected to the operative portion, and regulated to rotate around an axis of the tubular lumen insertion device. The cylindrical body is in contact with the main body, and is arranged to rotate integrally with the main body. Upon manually rotating the cylindrical body around its axis, the torque regulation mechanism prevents the torque transmission from the cylindrical body to the tubular insertion device by releasing the integral rotation between the cylindrical body and the main body if the cylindrical body is applied more than the predetermined torque.

The cylindrical body has a cylindrical distal end portion located at the side where the tubular lumen insertion device is located, and having a cylindrical proximal end located opposite to the side where the tubular lumen insertion device is located. The torque regulation mechanism is placed either at the contact portion between the main body and the cylindrical distal end portion or at the contact portion between the main body and the cylindrical proximal end.

The torque regulation mechanism has a body-side tooth portions which have at least two slope surfaces on the main body, and having a cylinder-side tooth portions which have at least two slope surfaces on the cylindrical body to mesh with the body-side tooth portions.

Each of the body-side tooth portions and the cylinder-side tooth portions incline the slope surfaces at different gradient angles depending upon situations in which the cylindrical body are rotated in one direction with the one slope surface, and rotated in another direction with the other slope surface.

The main body has at least one body-side projection at its outer peripheral portion, while at the same time, the cylindrical body has at least one cylinder-side projection at its outer peripheral portion so as to form a series of projections.

The tubular lumen insertion device, which is attached to the medical equipment, has a distal end portion formed by a metallic body, and having a shaft portion at least partly formed by a helical coil body which is provided by at least one of winding a metallic wire and stranding metallic wires.

In another embodiment of the invention, there is provided a medical equipment in which the main body has a first body end located at a side where the distal end portion of the tubular lumen insertion device is located and a second body end located opposite to the side where the distal end portion of the tubular lumen insertion device is located. The cylindrical body has the first cylindrical end configured to be in contact with the first body end, and a second cylindrical end configured to be in contact with the second body end.

The cylindrical body is arranged to be axially movable so as to bring either the first body end or the second body end into contact with the main body. The torque regulating mechanism has a first torque regulation portion and a second torque regulation portion. The first torque regulation portion is provided at a contact portion between the first body end and the first cylindrical end, and the second torque regulation portion is provided at a contact portion between the second body end and the second cylindrical end.

Such is the structure that the torque needed to release the integral rotation at the first torque regulation portion is different in magnitude from the torque needed to release the integral rotation at the second torque regulation portion.

In further embodiment of the invention, there is provided a tubular lumen insertion device which has a flexible and axially elongate body to be inserted into the tract from a distal end portion of the tubular lumen insertion device.

The tubular lumen insertion device has a shaft portion, an operative portion, a distal end portion and a medical equipment. The shaft portion is at least partly formed by a helical coil body which is provided by at least one of winding a metallic wire and stranding metallic wires. The operative portion is rotatably provided at a proximal end of the tubular lumen insertion device.

The medical equipment is provided to transmit the rotational movement of the operative portion to the shaft portion. The medical equipment has a cylindrical body in contact with the main body, and is arranged to rotate integrally with the main body.

The torque regulation mechanism is provided to block the integral rotation of the main body and the cylindrical body so as to render the torque incapable to transmit from the cylindrical body to the main body when the cylindrical body is applied more than the predetermined torque.

In this situation, the main body of the medical equipment has a first body end located at the distal end portion of the tubular lumen insertion device and a second body end located opposite to the distal end portion of the tubular lumen insertion device.

The cylindrical body has a first cylindrical end configured to be in contact with the first body end, and a second cylindrical end configured to be in contact with the second body end. The cylindrical body is arranged to be axially movable so as to bring either the first cylindrical end or the second body end into contact with the main body.

The torque regulating mechanism has a first torque regulation portion and a second torque regulation portion. The first torque regulation portion is provided at the contact portion between the first body end and the first cylindrical end, and the second torque regulation portion provided at the contact portion between the second body end and the second cylindrical end.

The torque regulation mechanism has body-side tooth portions which have at least two slope surfaces on the main body, and having a cylinder-side tooth portions which have at least two slope surfaces on the cylindrical body to mesh with the body-side tooth portions.

The body-side tooth portions and the cylinder-side tooth portions incline the slope surface at different gradient angles depending upon situations in which the cylindrical body are rotated in one direction to be in contact with the one slope surface, and rotated in another direction to be in contact with the other slope surface.

This enables the operator to distinguish the torque needed to release the integral rotation at the first torque regulation portion from the torque needed to release the integral rotation at the second torque regulation portion.

To describe the embodiment in a more specific manner, the structures of the medical equipment and tubular insertion device are explained in detail by referring to the accompanied drawings.

In the following description of the depicted embodiments, the same reference numerals are used for features of the same type.

### FIRST EMBODIMENT OF THE INVENTION

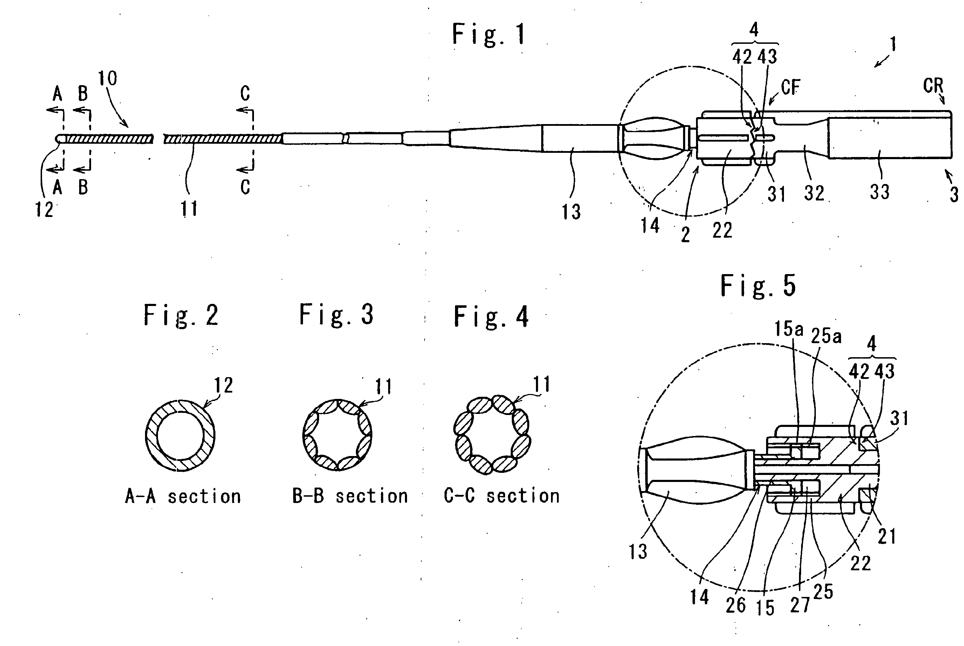

Referring to Figs. 1-10 which structurally show a medical equipment 1 according to a first embodiment of the invention, the medical equipment 1 is to be attached to a proximal end portion (right side shown) of an occlusion catheter 10 which serves as a tubular insertion device (tubular lumen insertion device). The occlusion catheter 10 has a shaft portion 11, a part of which forms a helical coil by at least one of winding a metallic wire and stranding metallic wires. The shaft portion 11 has a distal end portion 12 formed by a metallic body at a left end, and having an operative portion 13 at a proximal end of the shaft portion 11 as shown in Fig. 1.

To be specific, the shaft portion 11 forms a helical coil body. The helical coil body comprises a stranded coil made by closely stranding multiple pieces of a long, thin wire at a constant pitch of approx. Each wire comprising a helical coil body is made of a stainless steel wire. The material for wires is not all limited to stainless steel used in this example.

The wire can be made of any flexible metal material that has been traditionally used to produce catheter components. For example, a super-elastic alloy such as Ni-Ti based alloy or the like may be employed alone or in combination with the stainless steel.

In addition, the number of wires can be determined freely as deemed appropriate in accordance with, for example, the balance of flexibility and the rigidity expected of the helical coil body. For example, the helical coil body may be formed by stranding six to sixteen wires (0.075-0.20 mm in dia.), or more preferably by stranding 8 to 12 wires. For example, the helical coil body may be stranded with a lead pitch as 1.70-1.90 mm.

In the first embodiment of the invention, eight wires are employed (0.18 mm in dia.) with the lead pitch as 1.78 mm. It is to be noted that the shaft portion 11 of the helical coil body may be formed by winding a single one wire or a plurality of metallic wires instead of stranding the metallic wires.

An outer surface of the distal end portion of the shaft portion 11 is tapered by means of grinding procedure or the like so that the distal end portion is to be diametrically reduced progressively as approaching forward as shown in Fig. 3.

In the meanwhile, an outer surface of the proximal end of the shaft portion 11 is undulated as shown in Fig. 4 because it is not ground.

The distal end portion 12 is provided by securing a metallic cylinder body to a front end of the shaft portion 11 which is a portion to be percutaneously inserted into the tract of human body. The metallic cylinder body of the distal end portion 12 is made of a radiopaque material such as gold, platinum, platinum-rhodium alloy or the like. The metallic cylinder body of the distal end portion 12 is made of platinum in the first embodiment of the invention. The outer surface of the metallic cylinder body of the distal end portion 12 is represented by the smooth surface as shown in Fig. 2.

The operative portion 13 has a proximal end portion which provides a cylindrical connector 14 to be connected to the medical equipment 1. The cylindrical connector 14 has a proximal end which forms a diameter-increased flange portion 15, an outer surface of which forms a threaded portion 15a.

The occlusion catheter 10 is manipulatively rotated at its proximal side around its axis to move forward and rearward depending upon the lead pitch of the helical coil body. Then, the occlusion catheter 10 is inserted into an occlusive portion which makes a dilatation device (e.g., balloon catheter) incapable to pass after permitting a guide wire to pass through. The occlusion catheter 10 serves to produce a passage for the dilatation device which is manipulatively used thereafter.

Generally, the occlusion catheter 10 measures 750-1530 mm in entire length, and having the shaft portion 11 which measures 350-1100 mm in length with inner and outer diameters in turn as 0.35-0.66 mm and 0.50-1.00 mm. The distal end portion 12 of the shaft portion 11 measures 0.5-1.0 mm in length with inner and outer diameters in turn as 0.30-0.50 mm and 0.50-1.00 mm.

In the first embodiment of the invention, the occlusion catheter 10 measures 1450 mm in entire length, and having the shaft portion 11 which measures 1050 mm in length with maximum inner and outer diameters in turn as 0.46 mm and 0.71 mm. The distal end portion 12 of the shaft portion 11 measures 1.0 mm in length with inner and outer diameters in turn as 0.40 mm and 0.62 mm. The length, inner and outer diameters of the occlusion catheter 10 may be appropriately determined as desired depending upon the purpose to be applied.

The medical equipment 1 has a torque regulation mechanism 4 provided to block a torque transmission to the occlusion catheter 10. The medical equipment 1 has a main body 2 fixed to the cylindrical connector 14 not to relatively rotate to the occlusion catheter 10 in an axis thereof, and having a cylindrical body 3 brought into contact with the main body 2 to implement an integral rotation in unison with the main body 2.

The structure is such that the cylindrical body 3 is released from the integral rotation with the main body 2 so as to block a torque transmission to the occlusion catheter 10 when the cylindrical body 3 is applied more than a predetermined torque upon holding the cylindrical body 3 to rotate it around its axis.

Upon describing the medical equipment 1, a front end portion designates the left side of the medical equipment 1 as a side of the tubular insertion device in the drawings, and a rear end portion designates the right side of the occlusion catheter 10 as an opposite side of the tubular insertion device in the drawings.

The main body 2 is cylindrical in shape, and has an elongate bar portion 21 and having a diameter-increased portion 22 integrally defined on the front end of the elongate bar portion 21 to be diametrically larger than the elongate bar portion 21. The rear end of the diameter-increased portion 22 serves as a contact surface against the cylindrical body 3. The main body 2 has a stopper 23 screwed into the rear end of the elongate bar portion 21. It is to be noted that the main body 2 has a lumen 24, through which a guide wire (not shown) is inserted.

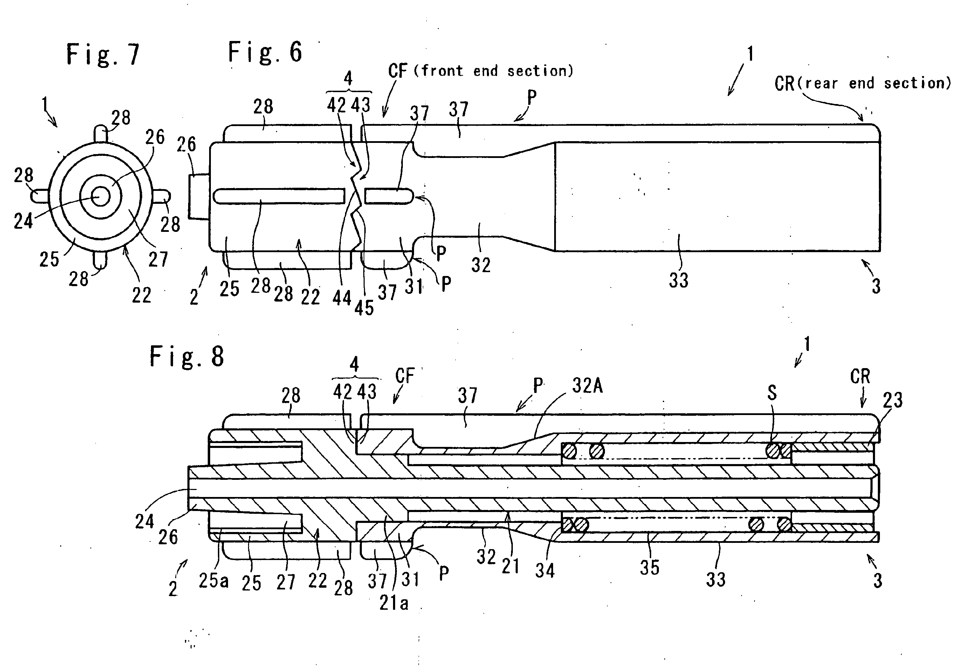

The elongate bar portion 21 has an intermediary portion 21a at a border with the diameter-increased portion 22. An outer diameter of the intermediary portion 21a is smaller than that of the diameter-increased portion 22, and is slightly smaller than an inner diameter of the front end of the cylindrical body 3 as shown in Fig. 8.

At the rear side of the intermediary portion 21a, the elongate bar portion 21 has an equi-diametrical dimension determined to be smaller than the diameter of the intermediary portion 21a along an axial direction of the elongate bar portion 21.

A front end of the diameter-increased portion 22 has an outer cylinder portion 25, an inner surface of which forms a threaded portion 25a. Within the outer cylinder portion 25, a middle cylinder portion 26 integrally extends concentrically toward the front end of the.diameter-increased portion 22. A front end of the middle cylinder portion 26 extends somewhat beyond the front end of the outer cylinder portion 25 with a annular space 27 defined between the middle cylinder portion 26 and the outer cylinder portion 25.

Upon connecting the main body 2 to the occlusion catheter 10 as shown in Fig. 5, the threaded portion 15a of the flange portion 15 is tightened into the threaded portion 25a of the outer cylinder portion 25 by inserting the cylindrical connector 14 into the middle cylinder portion 26. The main body 2 is inserted into the cylindrical body 3 in the rear end side of the diameter-increased portion 22. The cylindrical body 3 has a front end section (first body end) CF located in the side of the insertion device, and having a rear end section (second body end) CR located opposite to the side of the insertion device. Into the cylindrical body 3, the main body 2 except for the diameter-increased portion 22 is inserted. At the front end section CF, is an engagement cylinder 31 provided which is diametrically similar to the diameter-increased portion 22, so as to make the front end of the cylindrical body 3 contact with the rear end of the diameter-increased portion 22 as a two-side contact.

From the engagement cylinder 31 to the rear end section CR, the cylindrical body 3 has a constricted portion 32 and a handle portion 33 respectively. The constricted portion 32 is thinner than the engagement cylinder 31, and the handle portion 33 has a tapered portion 32A which diametrically increases progressively from the constricted portion 32 rearward.

Upon holding the cylindrical body 3, it enables the operator to pick the constricted portion 32 with the fingers and grasp the handle portion 33. The cylindrical body 3 has an inner wall diametrically enlarged from a stepped portion 34 rearward which is provided at the rear end of the tapered portion 32A, so as to form an enlarged bore 35.

The cylindrical body 3 is pushed toward the diameter-increased portion 22 by means of a helical spring S, so as to make the front end of the cylindrical body 3 contact with the rear end of the diameter-increased portion 22. The helical spring S is disposed around an outer surface of the elongate bar portion 21 and located within the enlarged bore 35.

In this situation, the helical spring S contacts its front end with the stepped portion 34 in its front end, and contacts with a front end of the stopper 23 in its rear end. By axially moving the stopper 23, it is possible to adjust a contractile and tensile length of t.he helical spring S as desired.

In the torque regulation mechanism 4 which is placed at a contact portion (two-side contact) between the main body 2 and the front end section CF, there are provided body-side tooth portions 42 and cylinder-side tooth portions 43 which are in turn provided at the main body 2 and the cylindrical body 3. The torque regulation mechanism 4 alternately engages the body-side tooth portions 42 with the cylinder-side tooth portions 43.

In this embodiment, there are 8 tooth portions 42, 43 respectively but at least one tooth portion, preferably 3 to 12 tooth portions can be provided.

In more tangible terms, the body-side tooth portions 42 is formed on an opposed end surface (rear end surface) of the diameter-increased portion 22 facing a front end surface of the cylindrical body 3. The cylinder-side tooth portions 43 is formed on an opposed end surface (front end surface) of the cylindrical body 3 facing the rear end surface of the diameter-increased portion 22, so as to serve as the torque regulation mechanism 4 at the contact portion between the main body 2 and the front end section CF.

Each of the body-side tooth portions 42 and the cylinder-side tooth portions 43 forms a triangular structure (non-equilateral triangle) defined by slope surfaces 44, 45. The slope surfaces 44, 45 have gradient angles differently represented so that the slope surface 44, 44 of the body-side tooth portion 42 and the cylinder-side tooth portion 43 tightly contact with each other in one rotational direction, and to the contrary, the slope surface 45, 45 tightly contact with each other in the opposite rotational direction.

The slope surface 44 inclines within the range of 9-45 degrees (preferably 15 degrees), and the slope surface 45 inclines at 9 degrees or greater but less than or equal to 90 degrees (preferably 30 degrees). The body-side tooth portions 42 and the cylinder-side tooth portions 43 forms eight teeth along a circular line at regular intervals, and each has a tooth height within the range of 0.3-1.5 mm (preferably 0.5 mm).

With the pushing force of the helical spring S and the inclination of the slope surface 44, 45 of the body-side tooth portion 42 and the cylinder-side tooth portion 43 determine an engaging force necessary between the body-side tooth portions 42 and the cylinder-side tooth portions 43 upon implementing an integral rotation of the main body 2 and the cylindrical body 3. When the engaging force is more than the torque applied to the cylindrical body 3, the torque releases the engagement between the cylinder-side tooth portions 43 and the body-side tooth portions 42. The structural characteristics, material property or the like of the occlusion catheter 10 predetermines the engaging force necessary between the tooth portions 42, 43 to such a degree as not to induce the torsional buckling on the occlusion catheter 10.

In this situation, when the cylindrical body 3 is applied more than the predetermined torque upon holding the cylindrical body 3 to rotate it around its axis, the torque releases an integral rotation between the cylinder-side tooth portions 43 and the body-side tooth portions 42, so as to make the cylindrical body 3 rotate in relative to the main body 2, thereby blocking the torque transmission to the occlusion catheter 10.

The main body 2 has body-side projections 28 extended outward and axially in an outer surface of the diameter-increased portion 22. Four body-side projections 28 are provided along the outer surface of the diameter-increased portion 22 at regular intervals (90 degrees).

The cylindrical body 3 has cylinder-side projections 37 extended outward and axially in an outer surface of the engagement cylinder 31. Four cylinder-side projections 37 are provided along the outer surface of the engagement cylinder 31 at regular intervals (90 degrees). One of the four cylinder-side projections 37 axially extends longer than the rest of the projections 37 so as to reach the handle portion 33 through the constricted portion 32. The cylinder-side projections 37 are adapted to forms a series of projections P aligned to the corresponding body-side projections 28.

It is to be noted that the main body 2, the cylindrical body 3 and the stopper 23 are each formed from an abrasion-durable polyacetal by means of an injection mold. Instead of the polyacetal, plastic material such as polycarbonate, polysulfone, polyethylene, polypropylene, polyamide or the like may be used. Instead of the injection mold, the main body 2, the cylindrical body 3 and the stopper 23 may be shaped by means of a cutting procedure or the like.

With the use of the medical equipment 1 and the tubular insertion device, to which the medical equipment 1 is attached, the blood vessel, the digestive tract, the urethra or the like in the human body are therapeutically treated or diagnosed.

In this instance, following procedures are taken upon dilating the cardiovascular occlusive area with the occlusion catheter 10 as the tubular insertion device (lumen insertion device).

The guide wire is percutaneously inserted to pass through the cardiovascular occlusion area in order to provide the occlusion area with an opening passage (not shown). Upon inserting the guide wire, the occlusion area permits the guide wire to pass a portion of the occlusion area in relatively soft area. Then, the medical equipment 1 is attached to the cylindrical connector 14 of the operative portion 13 of the occlusion catheter 10 (referred to Fig. 1).

A proximal end portion opposite to the distal end portion of the guide wire inserted into the cardiovascular system, is inserted to the occlusion catheter 10 and the main body 2 (lumen 24). The occlusion catheter 10 is inserted into the cardiovascular system along the guide wire under the fluoroscopic observation. The distal end portion 12 of the occlusion catheter 10 inserts to the occlusion area by observing the radiopaque distal end portion 12.

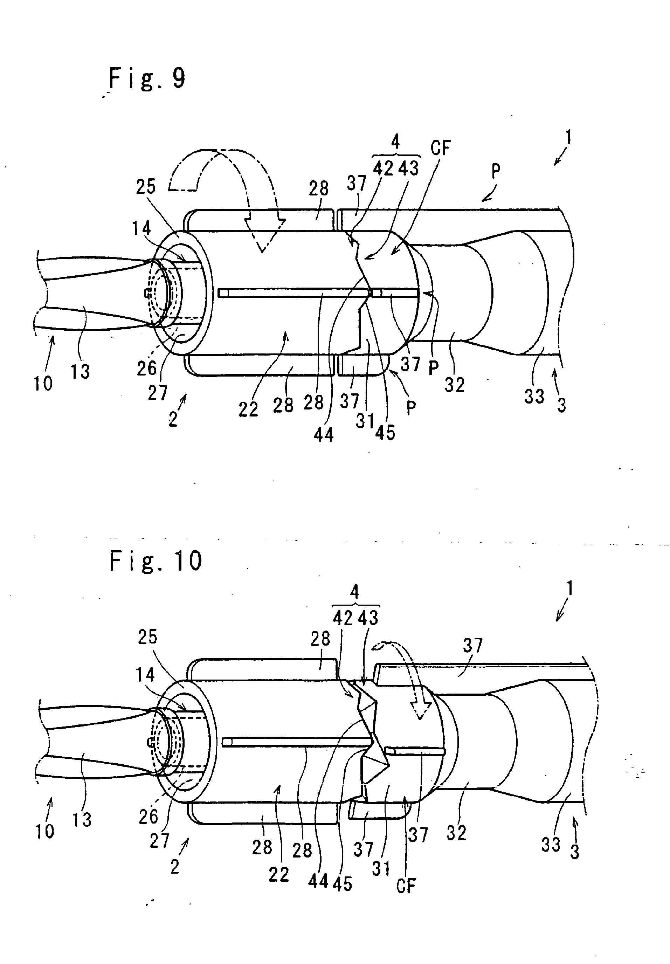

When the distal end portion 12 of the occlusion catheter 10 reaches the occlusion area, the cylindrical body 3 of the medical equipment 1 located at the proximal side of the occlusion catheter 10 is held to be operatively rotated with the cylindrical body 3 pushed frontward. Thus the torque applied to the cylindrical body 3 is transmitted to the main body 2 through the body-side tooth portions 42 and the cylinder-side tooth portions 43 as shown in Fig. 9.

This makes it possible to implement the integral rotation between the cylindrical body 3 and the main body 2, thereby advancing the occlusion catheter 10 into the cardiovascular system (human tract) in accordance with the lead pitch of the shaft portion (wire-stranded helical body) 11 , so as to insert the distal end portion 12 into the opening passage of the occlusion area along the guide wire.

This enables the operator to lead the distal end portion 12 and a leading end portion of the shaft portion 11 into the opening passage, thereby widening the opening passage to such a degree as to be substantially equal to the diameter of the leading end portion of the shaft portion 11.

After withdrawing the occlusion catheter 10 from the cardiovascular system, the balloon catheter is percutaneously inserted into the cardiovascular system along the guide wire if necessary. The balloon catheter further dilates the opening passage, and places a stent (not shown) to positively maintain the dilational opening passage.

When integrally rotating the cylindrical body 3 in unison with the main body 2 and the occlusion catheter 10, the cylinder-side projections 37 and the body-side projections 28 are integrally rotated as the series of the projections P formed in the axial direction.

By setting the series of the projections P as a "zero-position" before rotating the cylindrical body 3 (non-load applied), the "zero-position" enables the operator to recognize a single rotation each time when the "zero-position" comes around, so as to count the number of the rotations.

Based on the lead pitch of the shaft portion 11 provided on the wire-stranded helical coil body in the occlusion catheter 10, it is possible to calculate the feed per rotation of the occlusion catheter 10 when the operator recognizes the single rotation, thus enabling the operator to measure the length of the occlusion area by counting the number of the rotations.

Upon inserting the occlusion catheter 10 in the blood vessel or inserting it into the occlusion area, the distal end portion 12 or a part of occlusion catheter 10 encounters the vascular wall or the occlusion area. The encounter prevents the smooth rotation of the occlusion catheter 10. If the operator keeps the rotation even in the situation, the torque applied to the cylindrical body 3 may increase to more than a predetermined value.

In this instance, the torque causes to a slip between the body-side tooth portions 42 and the cylinder-side tooth portions 43 so as to rotationally displace therebetween that the cylindrical body 3 idles to the main body 2, thereby blocking the torque transmission to the main body 2 and the occlusion catheter 10 as shown in Fig. 10.

This enables the operator to have tactile feelings, which are an axial tactile feeling and a rotational tactile feeling, so as to perceive the torque more than the predetermined value.

The axial tactile feeling is recognized when pairs of meshing teeth slip out each other along the slope surfaces 44, 45 to create a small distance in the axial direction between them just before the teeth displace to the adjoining teeth for the idle rotation.

The rotational tactile feeling is recognized when the teeth slip over a plurality of facing teeth between the body-side tooth potion 42 and the cylinder-side tooth portion 43 during the idle rotation. This produces the tactile feelings to enable the operator to instantaneously perceive the torque more than the predetermined value (excessive torque).

During the idle rotation of the cylindrical body 3 relative to the main body 2, the cylinder-side tooth portions 43 collide against the body-side tooth portions 42. This produces the audible sound to enable the operator to instantaneously perceive the torque more than the predetermined value (excessive torque).

Particularly due to the sensitive axial tactile feeling (buoyant feeling) caused by slipping apart from each pairs of the teeth. The slight axial tactile feeling enables the operator to perceive an important warning message before giving an unnecessary burden to the patient to be therapeutically treated or diagnosed.

In association with the rotational movement of the cylindrical body 3 in relative to the main body 2, the cylinder-side tooth portions 43 rotationally moves in relative to the body-side tooth portions 42, so as to cancel the formation represented by the series of the projections P formed by the cylinder-side projection 37 and the body-side projection 28. This enables the operator to instantly perceive the generation of the torque more than the predetermined value.

This also makes the operator visually recognize the disarranged formation represented by the series of the projections P formed by the cylinder-side projection 37 and the body-side projection 28, so as to enable the operator to once more perceive that the torque is more than the predetermined value.

By providing the cylinder-side projections 37 and the body-side projections 28 with eyemarks (colors, markers or the like), it becomes more effective for the operator to visually confirm the generation of the torque more than the predetermined value.

With the structure aforementioned, it is possible for the operator to instantaneously perceive that the torque exceeds the predetermined value, and thereby preventing an unnecessary burden from being imposed on the patient's body.

Once the operator perceives the generation of the surplus torque, the operator pulls occlusion catheter 10 toward the outside of the body from the cardiovascular system with reversely rotating the occlusion catheter 10 in the opposite direction from the direction in which the occlusion catheter 10 is inserted to immediately solve causes which brought the torque more than the predetermined value. Upon solving one.of the causes, for example, the distal end portion 12 of the occlusion catheter 10 stuck in the occlusion area is drawn from before resuming to insert the occlusion catheter 10.

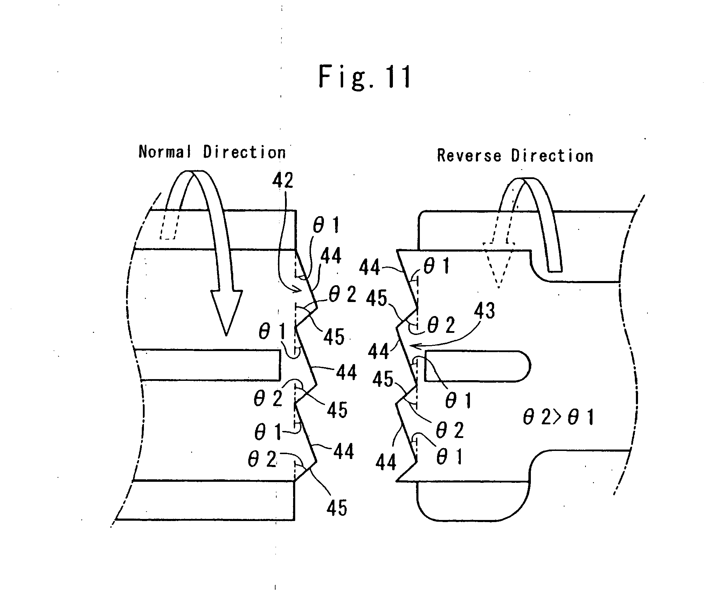

In this embodiment, the normal rotational direction is defined as the rotational direction in which the occlusion catheter 10 is inserted into the body, and angles (θ 1), (θ 2) are formed between a surface perpendicular to the rotational axis and each of the slope surfaces 44, 45.
This angular relationship between a gradient angles (θ 1), (θ 2) of the slope surface 44, 45 is determined as shown in Fig. 11. In this case, the angle (θ 1) is smaller than the angle (θ 2)

This means that the torque applied to the cylindrical body 3 in the reverse rotational direction is greater than the torque applied in the normal rotational direction. When rotating the cylindrical body 3 in the reverse rotational direction to relief the state that the occlusion catheter 10 is stuck in the occlusion area or the like with drawing the occlusion catheter 10 rearward, the operator can transmit a greater torque to the occlusion catheter 10.

The greater torque transmission serves a quick rotation of the cylindrical body 3 to draw the occlusion catheter 10. This enables a delicate operation for the therapeutic diagnosis or treated in the normal rotational direction, and enables a quick operation in the reverse rotational direction for the rearward movement of the occlusion catheter 10.

The medical equipment 1 with the torque regulation mechanism 4 also avoids the procedural delay caused from withdrawing the buckled catheter from the vascular system to exchange new one unlike the conventional catheter that may be subjected to the buckling due to the torque more than the predetermined value (a.k.a., excessive torque) when operatively rotating the catheter.

Namely, The medical equipment 1 with the torque regulation mechanism 4 prevents the surplus torque so as to continuously operate the occlusion catheter 10 without inviting the buckling on it, thereby obviating the necessity of exchanging the occlusion catheter 10.

Especially when the tubular insertion device is formed by the helical coil body such as the occlusion catheter 10, the occlusion catheter 10 has a good flexibility and torque transmissibility, but represents the undulating outer surface caused from the helical coil elements, and giving a chance that the occlusion area or the like likely lies partly on the undulating outer surface when inserting the catheter 10 into the occlusion area.

This imposes the excessive torque on the insertion device so as to likely cause the buckling on the insertion device to appear.

With the use of the medical equipment 1, it is possible to prevent the excessive torque from transmitting to the insertion device, so as not to induce the buckling on the insertion device. By taking the procedures to avoid the cause of the buckling, these procedures enable the operator to continue the operation with the good flexibility and torque transmissibility maintained when the insertion device is formed by the helical coil body.

This obviates the exchange of the insertion devices, thus enabling the operator to continue the operation safely by instantly perceiving the torque more than the predetermined value.

It is to be noted that although the meshing contact between the main body 2 and the front end section CF represents the contact between the rear end surface of the diameter-increased portion 22 and the front end surface of the cylindrical body 3, an inner surface of the main body 2 may come in contact with an outer surface of the front end section CF and vice versa.

Instead of the cylindrical body 3 pushed forward by means of the helical spring S to make the front end surface (cylindrical body 3) contact with the rear end surface (diameter-increased portion 22), the cylindrical body 3 may be pushed rearward by means of the helical spring S by placing the torque regulation mechanism 4 between the main body 2 and the rear end section CR which locates opposite to the insertion device.

### SECOND EMBODIMENT OF THE INVENTION

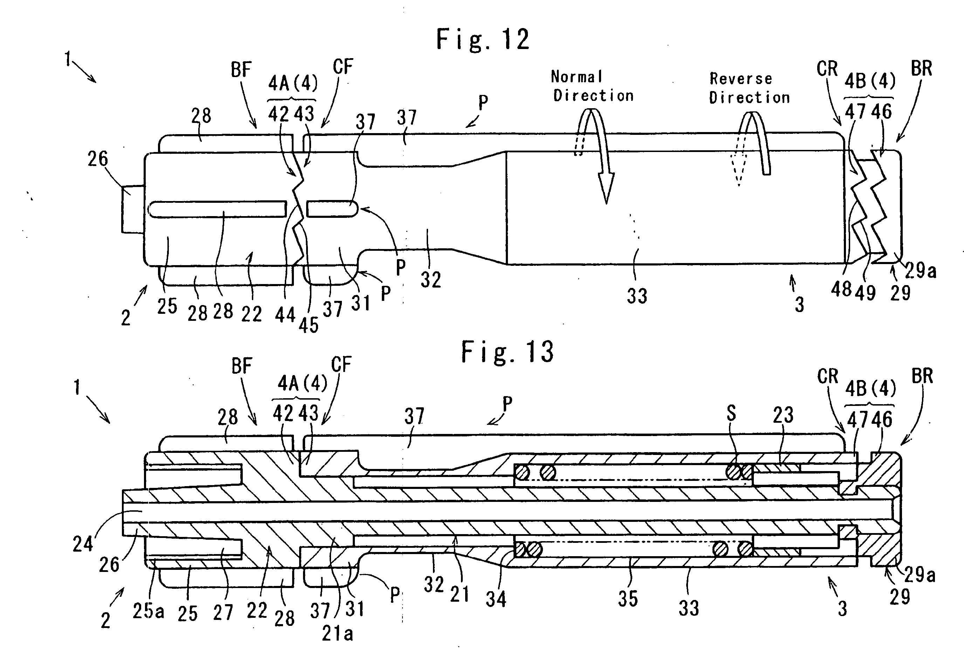

Figs. 12-14 show a second embodiment of the invention. In the second embodiment, structures and configurations merely different from those of the first embodiment are described herein. The main body 2 has a body front section (first cylindrical end) BF located at the side where the insertion device is located, and having a body rear section (second cylindrical end) BR located opposite side where the insertion device is located.

The body front section BF is adapted to come in contact with the cylindrical body front end section CF, and the body rear section BR adapted to come in contact with the cylindrical body rear end section CR. The body front section BF integrally forms the diameter-increased portion 22 on the front end side of the bar portion 21.

The body rear section BR has a cylindrical interfit elongation 29 provided to the rear end portion of the bar portion 21 discretely from the bar portion 21 and the diameter-increased portion 22. An rear end of the interfit elongation 29 has an enlarged flange 29a expanded radially, and an outer diameter of the flange 29a is substantially equal to an outer diameter of the handle portion 33. A front end surface of the flange 29a faces a rear end surface of the cylindrical body 3.

It is predetermined that a distance between the front end surface of the flange 29a and the rear end surface of the diameter-increased portion 22 is greater than the axial length of the cylindrical body 3 as shown in Figs. 12 and 13. The cylindrical body 3 is adapted to axially move along the bar portion 21 between the interfit elongation 29 and the diameter-increased portion 22. In the usual condition, the helical spring S pushes the cylindrical body 3 against the diameter-increased portion 22 so as to make the front end surface of the cylindrical body 3 contact with the rear end surface of the diameter-increased portion 22.

In this case, the rear end surface of the cylindrical body 3 does not come in contact with the front end surface of the flange 29a. When pulling the cylindrical body 3 against the pushing force of the helical spring S toward the front end surface of the flange 29a, the rear end surface of the cylindrical body 3 comes in contact with the front end surface of the flange 29a, while at the same time, releasing the contact between the front end surface of the cylindrical body 3 and the rear end surface of the diameter-increased portion 22.

The torque regulation mechanism 4 has a first torque regulation portion 4A and a second torque regulation portion 4B. The first torque regulation portion 4A is provided at the contact portion between the body front section BF and the cylindrical body front end section CF, and on the other hand, the second torque regulation portion 4B provided at the contact portion between the body rear section BR and the cylindrical body rear end section CR.

To be specific, the body-side tooth portion 42 is provided at an opposed surface (rear end surface) of the diameter-increased portion 22 to the cylindrical body 3, and the cylinder-side tooth portion 43 provided at an opposed surface (front end surface) of the cylindrical body 3 to the diameter-increased portion 22, thus placing the first torque regulation portion 4A at the contact portion between the body front section BF and the cylindrical body front end section CF. The second torque regulation portion 4B is formed by providing body-side tooth portions 46 at the front end surface of the flange 29a and providing cylinder-side tooth portions 47 at the rear end surface of the cylindrical body rear end section CR.

This resultantly means that the second torque regulation portion 4B is arranged at the contact portion between the body rear section BR and the cylindrical body rear end section CR. The body-side tooth portions 42 and the cylinder-side tooth portions 43 are the same as aforementioned in the first embodiment of the invention.

Regarding the body-side tooth portions 46 and the cylinder-side tooth portions 47, these portions 46, 47 form a triangular configuration (non-equilateral triangle) having slope surfaces 48, 49 analogous to the respective slope surfaces 42, 43.

When rotating the cylindrical body 3 in the reverse rotational direction, the slope surface 49 which comes in contact with the mating slope surface 49, inclines at a different gradient angle from the slope surface 48 which comes in contact with the mating slope surface 48 when rotating the cylindrical body 3 in the normal rotational direction.

The torque needed to release the integral rotational contact between the tooth portions 46, 47 (second torque regulation portion 4B) is greater than the torque needed to release the integral rotational contact between the tooth portions 42, 43 (first torque regulation portion 4A).

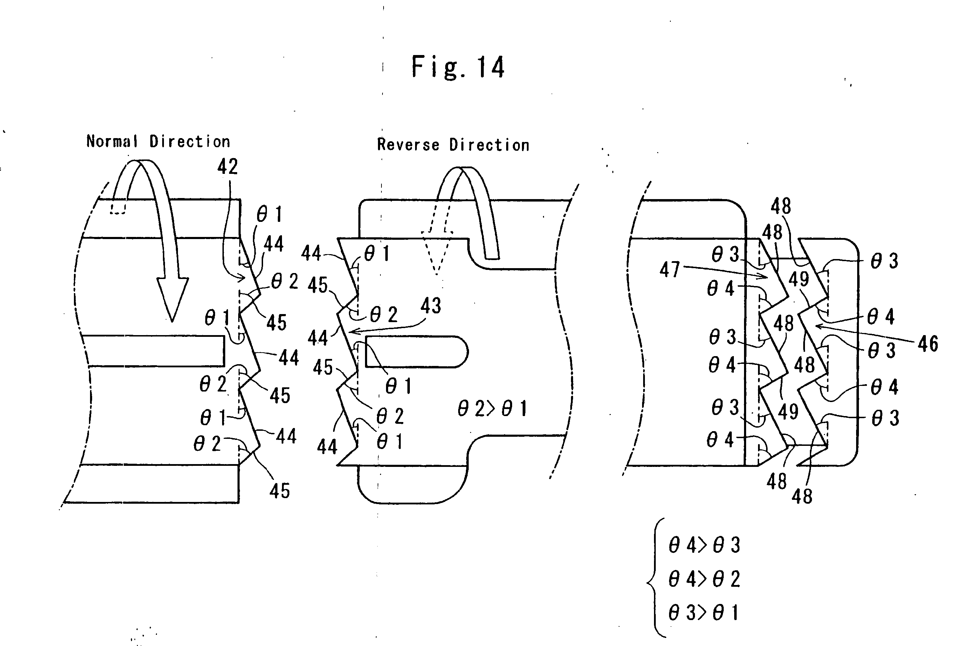

Namely, the slope surfaces 48, 49 (tooth portions 46, 47) have a tooth height and gradient angle (θ3, θ4) greater than the respective slope surfaces 44; 45 (tooth portions 42, 43) have the height and the gradient angle (θ1, θ2) as shown in Fig. 14. This means that the torque needed to release the integral rotational contact between the tooth portions 46, 47 is greater than the torque needed to release the integral rotational contact between the tooth portions 42, 43.

With the use of the medical equipment 1 and the occlusion catheter 10, to which medical equipment 1 is attached, following are procedures needed to dilate the cardiovascular occlusion area with emphases put on points different from the first embodiment of the invention.

The occlusion catheter 10 is operatively inserted into the cardiovascular system to reach the distal end portion 12 of the occlusion catheter 10 at the occlusion area in the same manner as described in the first embodiment of the invention.

The cylindrical body 3 of the medical equipment 1 located at the proximal side of the occlusion catheter 10 is held to be operatively rotated with the cylindrical body 3 push.ed frontward, the rotational operation transmits the torque of the cylindrical body 3 to the main body 2 through the body-side tooth portions 42 and the cylinder-side tooth portions 43 due to the reason that the cylindrical body 3 normally maintains its front side surface in contact with the rear end surface of the diameter-increased portion 22.

This makes it possible to integrally rotate the cylindrical body 3 in unison with the main body 2 and the occlusion catheter 10, thus advancing the catheter 10 toward the tract in accordance with the lead pitch of the shaft portion 11, so as to insert the distal end portion 12 into the opening passage of the occlusion area.

Upon introducing the occlusion catheter 10 into the blood vessel, so as to advance it into the occlusion area, the distal end portion 12 or a part of the occlusion catheter 10 encounters against the vascular wall or the occlusion area. The torque applied to the cylindrical body 3 may rise more than the predetermined value (excessive torque).

The excessive torque releases the engagement between the body-side tooth portions 42 and the cylinder-side tooth portions 43 in the first torque regulation portion 4A, thus permitting the cylindrical body 3 to rotate in relative to the main body 2 so as to resultantly block the torque transmission toward the occlusion catheter 10.

This enables the operator to have a tactile feeling so as to perceive the torque more than the predetermined value when one tooth overrides the others upon releasing the meshing contact between the body-side tooth portion 42 and the cylinder-side tooth portion 43.

This is true with the case in which one tooth overrides a plurality of teeth in succession to rotate the cylindrical body 3 against the main body 2, and the case in which the cylinder-side tooth portions 43 collide against the body-side tooth portions 42 to produce the audible sound upon rotating the cylindrical body 3 against the main body 2.

In association with the idle rotational movement of the cylindrical body 3 in relative to the main body 2, the cylinder-side tooth portions 43 rotationally moves in relative to the body-side tooth portions 42, so as to cancel the formation represented by the series of the projections P formed by the cylinder-side projections 37 and the body-side projection 28. This enables the operator to visually perceive the generation of the torque more than the predetermined value.

Once the operator perceives the tactile feeling, the operator pulls the occlusion catheter 10 toward the outside of the body from the cardiovascular system, with reversely rotating the occlusion catheter 10 in the opposite direction from the direction in which the occlusion catheter 10 is inserted to immediately solve causes which brought the torque more than the predetermined value.

In this case, the operator may pull the cylindrical body 3 rearward at the time of reversely rotating the cylindrical body 3. Upon pulling the cylindrical body 3 with the force more than the pushing force of the helical spring S, the pulling force releases the front end surface of the engagement cylinder 31 in the front end section CF from the rear end surface of the diameter-increased portion 22 and make the tear end surface of the cylindrical body 3 in the rear end section CR contact with the front end surface of the flange 29a, so as to engage the body-side tooth portions 46 with the cylinder-side tooth portions 47.

In this situation, the second torque regulation portion 4B controls to block the torque transmission from the cylindrical body 3 to the main body 2 when the torque is more than the predetermined value.

At the time of pulling the cylindrical body 3 rearward, the main procedures is to reversely rotate the occlusion catheter 10 to relief the state that the occlusion catheter 10 is stuck in the occlusion area or the like with drawing the occlusion catheter 10 toward the outside of the body.

Since the slope surfaces 48, 49 have the tooth height and gradient angle each greater than the respective slope surfaces 44, 45 have, the torque needed to release the integral rotational contact between the body-side tooth portions 46 and the cylinder-side tooth portions 47 is greater than the torque needed to release the integral rotational contact between the body-side tooth portions 42 and the cylinder-side tooth portions 43.

This makes it easy to transmit the torque upon holding the cylindrical body 3 to rotate it reversely, so as to efficiently implement the procedures needed to relief the state that the occlusion catheter 10 is stuck in the occlusion area or the like with drawing the occlusion catheter 10 rearward.

The procedures requires to pull the cylindrical body 3 against the pushing force of the helical spring S. The pushing force acts forward to release the integral rotational contact between the two tooth portions 46, 47, the operator feels some difficulty upon handling the medical equipment 1 unless the engagement force of the two tooth portions 46, 47 is strong enough to compensate the pushing force of the spring S so that the engagement force of the two tooth portions 46, 47 is defined greater than the engagement force of the body-side tooth portions 42 and the cylinder-side tooth portions 43.

The engagement force between the body-side tooth portions 46 and the cylinder-side tooth portions 47 is defined by angles (θ 3), ( θ 4) formed between a surface perpendicular to the rotational axis and each of the slope surfaces 48, 49.

As one example of the embodiment, the angle (θ 4) is greater than each of the angles (θ 2), (θ 3) and the angle (θ 3) is greater than the angles (θ 1) as shown in Fig. 14.

The above torque arrangement makes it easy to continue the procedures of rotating the cylindrical body 3, while at the same time, the operator pulls it regardless of its rotational direction (reverse or normal).

When rotating the cylindrical body 3 in the normal rotational direction, the slope surface 48 which comes in contact with the mating slope surface 48, inclines smaller than the slope surface 49 which comes in contact with the mating slope surface 49 when rotating the cylindrical body 3 in the reverse rotational direction.

In this case, the angle (θ 3) is smaller than the angle (θ 4).

Considering that the reverse direction of the cylindrical body 3 means to relief the state that the occlusion catheter 10 is stuck in the occlusion area or the like with drawing the occlusion catheter 10 rearward, it is advantageous to quickly rotate the cylindrical body 3 in the reverse rotational direction with an increased torque upon rotating the cylindrical body 3 with the pulling force applied rearward.

This enables the operator to a delicate handling upon rotating the cylindrical body 3 in the normal rotational direction, and enabling the operator to a quick operation upon rotating the cylindrical body 3 in the reverse rotational direction.

When rotating the cylindrical body 3 with the pulling force less than the pushing force of the helical spring S or without pulling the cylindrical body 3, this procedure permits the front end surface of the engagement cylinder 31 to be in contact with the rear end surface of the diameter-increased portion 22 so as to mesh the body-side tooth portions 42 with the cylinder-side tooth portions 43.

In this situation, the first torque regulation portion 4A controls to block the torque transmission from the cylindrical body 3 to the main body 2 when the torque is more than the predetermined value.

According to the second embodiment of the invention, when pulling the occlusion catheter 10 from the tract in the withdrawal direction to cause a disengagement between the front end surface of the engagement cylinder 31 and the rear end surface of the diameter-increased portion 22 upon pulling the cylindrical body 3 rearward, the pulling force permits the rear end surface of the cylindrical body 3 to be in contact with the front end surface of the flange 29a.

This permits the body-side tooth portions 46 to engage with the cylinder-side tooth portions 47 so that the cylindrical body 3 can promptly transmit the torque to the main body 2, thus enabling the operator to continue the procedures subsequently needed.

Namely, it is possible to effectively control the torque transmitted to the occlusion catheter 10 not merely upon inserting the occlusion catheter 10 into the tract but also upon pulling the occlusion catheter 10 out of the tract. This induces substantially no inequality on the torque transmitted from the cylindrical body 3 to the main body 2 during the use of the medical equipment 1.

### THIRD EMBODIMENT OF THE INVENTION

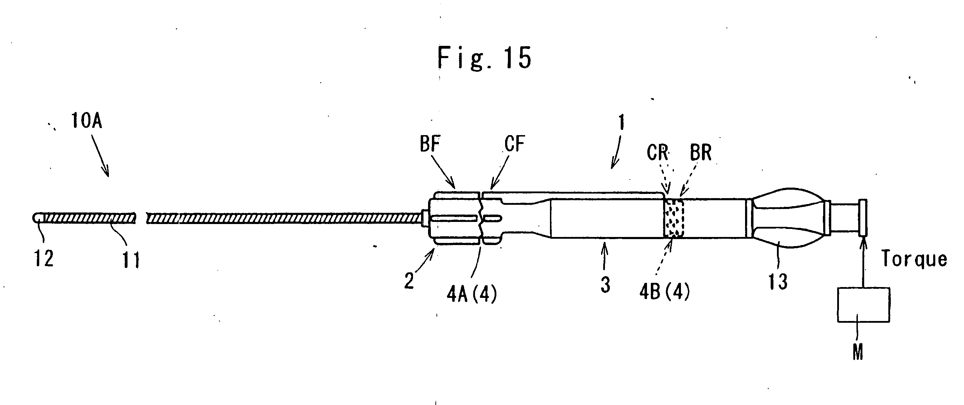

Fig. 15 show a third embodiment of the invention in which a tubular insertion device 10A is provided. The insertion device 10A has the distal end portion 12 provided at one end of the insertion device 10A, and the shaft portion 11 having the helical coil body at least partly formed by at least one of winding a metallic wire and stranding metallic wires.

At the other end of the insertion device 10A, the operative portion 13 is rotatably provided to position outside the human body to transmit the rotational movement of the operative portion 13 to the medical equipment 1.

The medical equipment 1 has the main body 2, the cylindrical body 3 and the torque regulation mechanism 4. The cylindrical body 3 engages with the main body 2 to integrally rotates in unison with the main body 2. In the same manner as described at the second embodiment of the invention, the torque regulation mechanism 4 is provided which releases the integral rotational contact between the cylindrical body 3 and the main body 2 so as to block the torque transmission from the cylindrical body 3 to the main body 2 when the torque is more than the predetermined value.

Although the first and second embodiment of the invention in which the medical equipment 1 is connected to the operative portion 13 of the insertion device, the main body 2 of the medical equipment 1 in this embodiment is connected to the shaft portion 11 and the cylindrical body 3 connected to the operative portion 13 to integrally rotate with the operative portion 13 as shown in Figs. 15.

In use, the insertion device 10A is percutaneously inserted at its shaft portion 11 and the distal end portion 12 into the tract with the medical equipment 1 and the operative portion 13 located outside the human body.

Upon rotating the insertion device 10A, the cylindrical body 3 or the operative portion 13 may be held and rotated. An electric motor M may be connected to the operative portion 13 to rotate it by means of the motor M.

### MODIFICATION FORMS

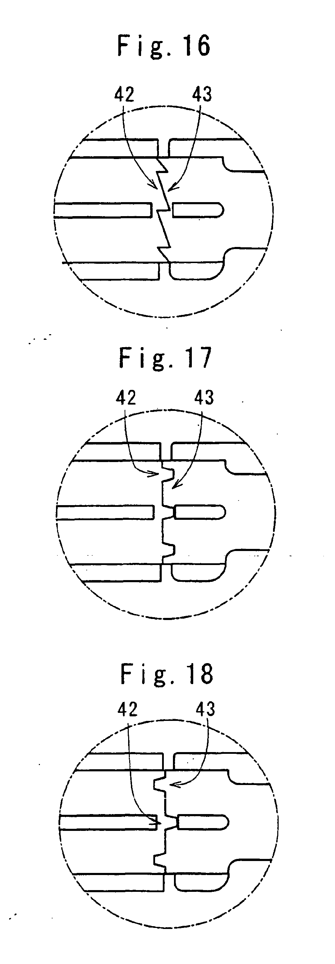

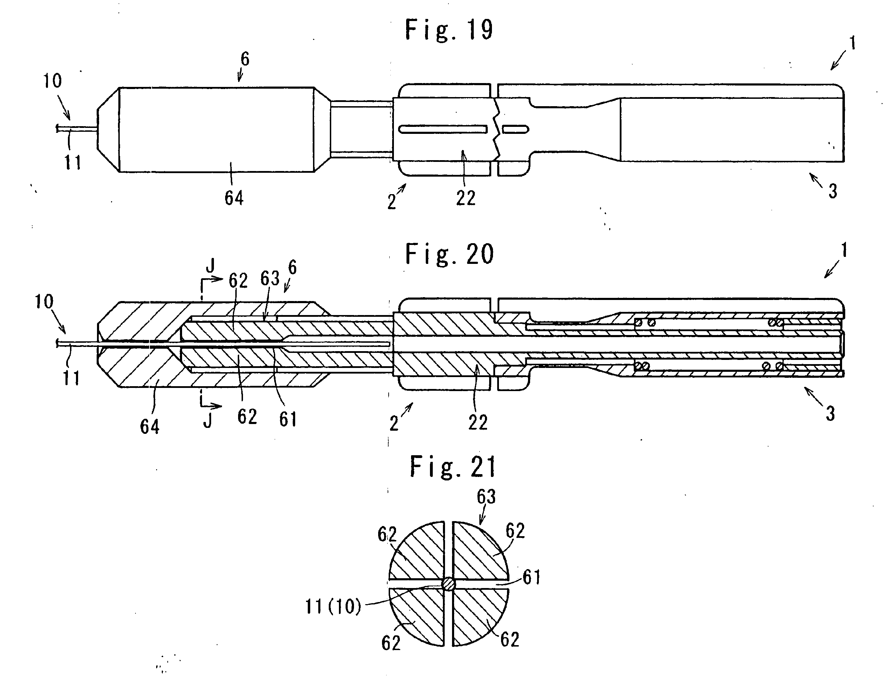

(a) Although, when rotating the cylindrical body 3 in the normal rotational direction, the slope surface 44 (48) which comes in contact with the mating slope surface 44 (48), inclines smaller than the slope surface 45 (49) which comes in contact with the mating slope surface 45 (49) when rotating the cylindrical body 3 in the reverse rotational direction in the first, second and third embodiment of the invention, the body-side tooth portions 42 (46) and the cylinder-side tooth portions 43 (47) may form an equilateral triangle instead of the non-equilateral triangle.
(b) Instead of the slope surface 44 (45) having the gradient angle of 9-45 degrees and more than 9 degrees but less than 90 degrees exclusive, one of the gradient angle in the slope surfaces 44, 45 may be 90 degrees to form a right-angled triangle as shown in Fig. 16.
(c) The body-side tooth portions 42 and the cylinder-side tooth portions 43 may be formed into a trapezoidal configuration by truncating apexes of the triangular teeth as shown in Fig. 17. The tooth breadth of the body-side tooth portions 42 may be different from that of the cylinder-side tooth portions 43. In the slope surfaces 44, 45, breadth-reduced teeth may be formed to protract forward and retract rearward alternately as shown in Fig. 18. The body-side tooth portions 46 and the cylinder-side tooth portions 47 may be formed in the same manner as mentioned above.
(d) Although the torque needed to release the integral rotational contact at the second torque regulation portion 4B is greater than the torque needed to release the integral rotational contact at the first torque regulation port ion 4A in the second and third embodiment of the invention, the torque needed at the second torque regulation portion 4B may be equal to the torque needed at the first torque regulation portion 4A.
(e) Although the connection between the main body 2 of the medical equipment 1 and the occlusion catheter 10 is done by fitting the threaded portion 15a of the flange portion 15 into the threaded portion 25a of the outer cylinder portion 25, with placing the cylindrical connector 14 in the annular space 27 and locating the middle cylinder portion 26 into the cylindrical connector 14, the connection between the main body 2 of the medical equipment 1 and the occlusion catheter 10 can use various means. By way of illustration, the connection may be done by means of a chuck 6 as shown in Figs. 19-21. In this situation, the chuck 6 has an inner clamp 63 including quadripartite claw elements 62 each opposed through a cruciform slit 61, and secured to the front end surface of the diameter-increased portion 22. An outer clamp 64 is provided which is to be interfit over the inner clamp 63. By setting the inner clamp 63 around the shaft portion 11, and inserting the outer clamp 64 over the inner clamp 63 to tighten the claw elements 62 against the shaft portion 11, it is possible to connect the shaft portion 11 to the main body 2.
(f) Instead of the occlusion catheter 10 used as the tubular insertion device in the first and second embodiment of the invention, a catheter, a guide wire, a balloon catheter and an atherectomy catheter et al. may be employed.
(g) Although the medical equipment 1 of the third embodiment of the invention is structurally similar to the medical equipment 1 of the second embodiment of the invention, it may be made similar to the medical equipment 1 of the first embodiment of the invention. Namely, the torque regulation mechanism 4 may be placed either at the contact portion between the main body 2 and the cylindrical body front end section CF or at the contact portion between the main body 2 and the cylindrical body rear end section CR.
(h) Instead of the torque regulation mechanism 4 having the body-side tooth portions 42 (46) and the cylinder-side tooth portions 43 (47), a frictional engagement structure or a magnetic engagement mechanism et al. may be used.

## Claims

1. A medical apparatus (1) comprising:
a main body (2) provided to be connected to a tubular insertion device (10) which is to be inserted inside a human body; and
a cylindrical body (3) provided in contact with said main body (2) and arranged integrally to rotate said main body with said tubular insertion device;
**characterised by**:
a torque regulating mechanism (4) located at a contact portion between said main body (2) and said cylindrical body (3);
said torque regulating mechanism (4) provided to rotationally displace said cylindrical body (3) to said main body at the contact portion to release an integral rotation between said cylindrical body and said main body so as to block a torque transmission to said tubular insertion device (10) when more than a predetermined torque is applied to said cylindrical body.

2. A medical apparatus according to claim 1, wherein said cylindrical body (3) has a first cylindrical body end located at a side where said tubular insertion device (10) is located and a second cylindrical body end located at a side different from the side where said tubular insertion device is located; and
said torque regulating mechanism (4) is located either at the contact portion between said main body and said first cylindrical body end or at the contact portion between said main body and said second cylindrical body end.

3. A medical apparatus according to claim 1, wherein said main body (2) has a first main body end (BF) located at a side where said tubular insertion device (10) is located and a second main body end (BR) located at a different side where said tubular insertion device is located;
said cylindrical body (3) having a first cylindrical body end (CF) configured to be in contact with said first main body end (BF), and a second cylindrical body end (CR) configured to be in contact with said second main body end (BR);
said cylindrical body (3) being arranged to be axially movable between said first main body end (BF) and said second main body end (BR); and
said torque regulating mechanism (4) having a first torque regulation portion (4A) and a second torque regulation portion (4B), said first torque regulation portion (4A) being provided at the contact portion between said first main body end (BF) and said first cylindrical body end (CF), and said second torque regulation portion (4B) being provided at the contact portion between said second main body end (BR) and said second cylindrical body end (CR).

4. A medical apparatus according to claim 3, wherein a torque needed to release said integral rotation at said first torque regulation portion (4A) is different from a torque needed to release said integral rotation at said second torque regulation portion (4B).

5. A medical apparatus according to claim 1, 2, 3 or 4, wherein said torque regulating mechanism includes a main body-side tooth portion (42) provided at said main body (2), and a cylindrical body-side tooth portion (43) provided at said cylindrical body (3) so as to mesh with said main body-side tooth portion (42).

6. A medical apparatus according to claim 5, wherein said main body-side tooth portion (42) and said cylindrical body-side tooth portion (43) respectively have at least one slope surface.

7. A medical apparatus according to claim 5, wherein said main body-side tooth portion (42) and said cylindrical body-side tooth portion (43) respectively have at least two slope surfaces of different angles.

8. A medical apparatus according to any one of the preceding claims, wherein an outer peripheral portion of said main body (2) has at least one main body-side projection (28), and
an outer peripheral portion of said cylindrical body (3) has at least one cylindrical body-side projection (37) which forms a series of projections with said main body-side projection.

9. A medical apparatus according to any one of the preceding claims, further comprising a tubular insertion device (10) selected from the group consisting of a catheter, a guide wire and a balloon catheter.

10. A medical apparatus according to any one of the preceding claims, comprising a tubular insertion device (10) having a distal end portion at one end formed by a metallic body, and a shaft portion at least partly having a helical coil body formed by at least one of winding a metallic wire and stranding metallic wires.

11. A medical apparatus according to claim 9 or 10, further comprising an electric motor to rotate said tubular insertion device.

## Patentansprüche

1. Medizinische Vorrichtung (1), welche umfasst:
einen dafür vorgesehenen Hauptkörper (2), mit einer rohrförmigen Einführvorrichtung (10), welche in einen menschlichen Körper einzuführen ist, verbunden zu werden, und
einen Zylinderkörper (3), der in Kontakt mit dem Hauptkörper (2) vorgesehen ist und integral angeordnet ist, um den Hauptkörper mit der rohrförmigen Einführvorrichtung zu drehen;
**gekennzeichnet durch**:
einen an einem Kontaktabschnitt zwischen dem Hauptkörper (2) und dem Zylinderkörper (3) angeordneten Drehmomentregelmechanismus (4);
wobei der Drehmomentregelmechanismus (4) vorgesehen ist, um zum Freigeben einer integralen Drehung zwischen dem Zylinderkörper und dem Hauptkörper den Zylinderkörper (3) an dem Kontaktabschnitt drehend zu dem Hauptkörper zu verschieben, um eine Drehmomentübertragung zu der rohrförmigen Einführvorrichtung (10) zu unterbinden, wenn mehr als ein vorbestimmtes Drehmoment auf den Zylinderkörper ausgeübt wird.

2. Medizinische Vorrichtung nach Anspruch 1, **dadurch gekennzeichnet, dass** der zylindrische Körper (3) ein erstes Zylinderkörperende, das sich an einer Seite befindet, an der sich die rohrförmige Einführvorrichtung (10) befindet, und ein zweites Zylinderkörperende, das sich an einer anderen Seite als der Seite, an der sich die rohrförmige Einführvorrichtung befindet, befindet, aufweist; und
sich der Drehmomentregelmechanismus (4) entweder an dem Kontaktabschnitt zwischen den Hauptkörper und dem ersten Zylinderkörperende oder an dem Kontaktabschnitt zwischen dem Hauptkörper und dem zweiten Zylinderkörperende befindet.

3. Medizinische Vorrichtung nach Anspruch 1, **dadurch gekennzeichnet, dass** der Hauptkörper (2) ein erstes Hauptkörperende (BF), welches sich an einer Seite befindet, an der sich die rohrförmige Einführvorrichtung (10) befindet, und ein zweites Hauptkörperende (BR), welches sich an einer anderen Seite als der, an der sich die rohrförmige Einführvorrichtung befindet, befindet, aufweist;
wobei der Zylinderkörper (3) ein dafür ausgelegtes erstes Zylinderkörperende (CP), mit dem ersten Hauptkörperende (BF) in Kontakt zu stehen, und ein dafür ausgelegtes zweites Zylinderkörperende (CR), mit dem zweiten Hauptkörperende (BR) in Kontakt zu stehen, aufweist;
wobei der Zylinderkörper (3) so angeordnet ist, dass er zwischen dem ersten Hauptkörperende (BF) und dem zweiten Hauptkörperende (BR) axial beweglich ist; und
der Drehmomentregelmechanismus (4) einen ersten Drehmomentregelabschnitt (4A) und einen zweiten Drehmomentregelabschnitt (4B) aufweist, wobei der erste Drehmomentregelabschnitt (4A) an dem Kontaktabschnitt zwischen dem ersten Hauptkörperende (BF) und dem ersten Zylinderkörperende (CF) vorgesehen ist und der zweite Drehmomentregelabschnitt (4B) an dem Kontaktabschnitt zwischen dem zweiten Hauptkörperende (BR) und dem zweiten Zylinderkörperende (CR) vorgesehen ist.

4. Medizinische Vorrichtung nach Anspruch 3, **dadurch gekennzeichnet, dass** ein zum Freigeben der integralen Drehung an dem ersten Drehmomentregelabschnitt (4A) benötigtes Drehmoment sich von einem zum Freigeben der integralen Drehung an dem zweiten Drehmomentregelabschnitt (4B) benötigten Drehmoment unterscheidet.

5. Medizinische Vorrichtung nach Anspruch 1, 2, 3 oder 4, **dadurch gekennzeichnet, dass** der Drehmomentregelmechanismus einen an dem Hauptkörper (2) vorgesehenen hauptkörperseitigen Zahnabschnitt (42) und einen an dem Zylinderkörper (3) zum Eingriff mit dem hauptkörperseitigen Zahnabschnitt (42) vorgesehenen zylinderkörperseitigen Zahnabschnitt (43) umfasst.

6. Medizinische Vorrichtung nach Anspruch 5, **dadurch gekennzeichnet, dass** der hauptkörperseitige Zahnabschnitt (42) und der zylinderkörperseitige Zahnabschnitt (43) jeweils mindestens eine Neigungsfläche aufweisen.

7. Medizinische Vorrichtung nach Anspruch 5, **dadurch gekennzeichnet, dass** der hauptkörperseitige Zahnabschnitt (42) und der zylinderkörperseitige Zahnabschnitt (43) jeweils mindestens zwei Neigungsflächen mit verschiedenen Winkeln aufweisen.

8. Medizinische Vorrichtung nach einem der vorstehenden Ansprüche, **dadurch gekennzeichnet, dass** ein äußerer Umfangsabschnitt des Hauptkörpers (2) mindestens einen hauptkörperseitigen Vorsprung (28) aufweist und
ein äußerer Umfangsabschnitt des Zylinderkörpers (3) mindestens einen zylinderkörperseitigen Vorsprung (37) aufweist, der mit dem hauptkörperseitigen Vorsprung eine Reihe von Vorsprüngen bildet.

9. Medizinische Vorrichtung nach einem der vorstehenden Ansprüche, welche weiterhin eine aus der Gruppe bestehend aus einem Katheter, einem Führungsdraht und einem Ballonkatheter ausgewählte rohrförmige Einführvorrichtung (10) umfasst.

10. Medizinische Vorrichtung nach einem der vorstehenden Ansprüche, welche eine rohrförmige Einführvorrichtung (10), die einen durch einen metallischen Körper gebildeten distalen Endabschnitt an einem Ende aufweist, und einen Wellenabschnitt, der zumindest teilweise einen spiralförmigen Spulenkörper aufweist, der durch mindestens eines von Wickeln eines Metalldrahts und Flechten von Metalldrähten gebildet ist, umfasst.

11. Medizinische Vorrichtung nach Anspruch 9 oder 10, welche weiterhin einen Elektromotor zum Drehen der rohrförmigen Einführvorrichtung umfasst.

## Revendications

1. Appareil médical (1), comprenant :
un corps principal (2) disposé de façon à être relié à un dispositif d'insertion tubulaire (10) qui est destiné à être inséré à l'intérieur d'un corps humain ; et
un corps cylindrique (3) disposé en contact avec ledit corps principal (2) et configuré d'un seul tenant pour faire tourner ledit corps principal avec ledit dispositif d'insertion tubulaire ;
**caractérisé par** :
un mécanisme de régulation de couple (4) situé en une partie de contact entre ledit corps principal (2) et ledit corps cylindrique (3) ;
ledit mécanisme de régulation de couple (4) étant disposé de façon à déplacer en rotation ledit corps cylindrique (3) par rapport audit corps principal au niveau de la partie de contact pour libérer une rotation intégrée entre ledit corps cylindrique et ledit corps principal de façon à bloquer une transmission de couple audit dispositif d'insertion tubulaire (10) lorsqu'un couple supérieur à un couple prédéterminé est appliqué audit corps cylindrique.

2. Appareil médical selon la revendication 1, dans lequel ledit corps cylindrique (3) comporte une première extrémité de corps cylindrique située en un côté où est situé ledit dispositif d'insertion tubulaire (10) et une deuxième extrémité de corps cylindrique située en un côté différent du côté où est situé ledit dispositif d'insertion tubulaire ; et
ledit mécanisme de régulation de couple (4) est situé soit au niveau de la partie de contact entre ledit corps principal et ladite première extrémité de corps cylindrique soit au niveau de la partie de contact entre ledit corps principal et ladite deuxième extrémité de corps cylindrique.

3. Appareil médical selon la revendication 1, dans lequel ledit corps principal (2) comporte une première extrémité de corps principal (BF) située en un côté où est situé ledit dispositif d'insertion tubulaire (10) et une deuxième extrémité de corps principal (BR) située en un côté différent de celui où est situé ledit dispositif d'insertion tubulaire ;
ledit corps cylindrique (3) comportant une première extrémité de corps cylindrique (CF) configurée de façon à être en contact avec ladite première extrémité de corps principal (BF), et une deuxième extrémité de corps cylindrique (CR) configurée de façon à être en contact avec ladite deuxième extrémité de corps principal (BR) ;
ledit corps cylindrique (3) étant configuré de façon à être axialement mobile entre ladite première extrémité de corps principal (BF) et ladite deuxième extrémité de corps principal (BR) ; et
ledit mécanisme de régulation de couple (4) comportant une première partie de régulation de couple (4A) et une deuxième partie de régulation de couple (4B), ladite première partie de régulation de couple (4A) étant disposée au niveau de la partie de contact entre ladite première extrémité de corps principal (BF) et ladite première extrémité de corps cylindrique (CF), et ladite deuxième partie de régulation de couple (4B) étant disposée au niveau de la partie de contact entre ladite deuxième extrémité de corps principal (BR) et ladite deuxième extrémité de corps cylindrique (CR).

4. Appareil médical selon la revendication 3, dans lequel un couple nécessaire pour libérer ladite rotation intégrée au niveau de ladite première partie de régulation de couple (4A) est différent d'un couple nécessaire pour libérer ladite rotation intégrée au niveau de ladite deuxième partie de régulation de couple (4B).

5. Appareil médical selon la revendication 1, 2, 3 ou 4, dans lequel ledit mécanisme de régulation de couple comprend une partie de dent de côté de corps principal (42) disposée au niveau dudit corps principal (2), et une partie de dent de côté de corps cylindrique (43) disposée au niveau dudit corps cylindrique (3) de façon à s'engrener avec ladite partie de dent de côté de corps principal (42).

6. Appareil médical selon la revendication 5, dans lequel ladite partie de dent de côté de corps principal (42) et ladite partie de dent de côté de corps cylindrique (43) comportent respectivement au moins une surface en pente.

7. Appareil médical selon la revendication 5, dans lequel ladite partie de dent de côté de corps principal (42) et ladite partie de dent de côté de corps cylindrique (43) comportent respectivement au moins deux surfaces en pente d'angles différents.

8. Appareil médical selon l'une quelconque des revendications précédentes, dans lequel une partie périphérique extérieure dudit corps principal (2) comporte au moins une saillie de côté de corps principal (28), et
une partie périphérique extérieure dudit corps cylindrique (3) comporte au moins une saillie de côté de corps cylindrique (37) qui forme une série de saillies avec ladite saillie de côté de corps principal.

9. Appareil médical selon l'une quelconque des revendications précédentes, comprenant de plus un dispositif d'insertion tubulaire (10) sélectionné parmi le groupe comprenant un cathéter, un fil de guidage et un cathéter à ballonnet.

10. Appareil médical selon l'une quelconque des revendications précédentes, comprenant un dispositif d'insertion tubulaire (10) comportant une partie d'extrémité distale à une extrémité formée par un corps métallique, et une partie d'arbre comportant au moins partiellement un corps d'enroulement hélicoïdal formé par au moins l'un d'un enroulement d'un fil métallique et d'un tressage de fils métalliques.

11. Appareil médical selon la revendication 9 ou 10, comprenant de plus un moteur électrique pour faire tourner ledit dispositif d'insertion tubulaire.
